# EUROPEAN PATENT APPLICATION

(11) **EP 4 834 984 A1**
(43) Date of publication of application: **04.11.2026**
(21) Application number: 25173830.8
(22) Date of filing: 30.04.2025
(51) Int. Cl.: C08J 11/24, C07C 67/03

(54) **GLYCOLYSIS OF POLYETHYLENE TEREPHTHALATE**

(71) Applicant: The Provost, Fellows, Foundation Scholars, and the other members of Board, of the College of the Holy & Undiv. Trinity of Queen Elizabeth near Dublin, Dublin 2 (IE)
(72) Inventor: CONNON, Stephen, Dublin, D02R590 (IE); PEDRINI, Lorenzo, Dublin, D02R590 (IE); KENNY, Lauren, Dublin, D02R590 (IE); JAIN, Anshul, Dublin, D02R590 (IE)
(74) Representative: FRKelly

(57) **Abstract**

Methods for depolymerising polymers comprising polyethylene terephthalate units, such as polyethylene terephthalate, by glycolysis are disclosed.

## Description

### Field

The present invention is concerned with glycolysis of polymers comprising polyethylene terephthalate units, such as polyethylene terephthalate, using magnesium catalysts.

### Background

It has been estimated that from 1950 to 2015, 6.3 x 10¹² kg of plastics have been synthesised - with a recycle rate of just ca. 9%. It has further been estimated, that without intervention, by 2050 12 x 10¹³ kg of these polymers will either be in landfill or out polluting the natural world, therefore (until replacement of traditional plastics with sustainable materials is widespread) improvement in both the rate and efficiency of recycling is needed to reduce both the dependence on oil-derived virgin plastic and the contribution its synthesis/disposal makes to the current climatological and ecological emergency.

Poly(ethylene terephthalate) (PET) polyester is a major constituent in certain beverage bottles, textile fibres and food tray/pharmaceutical packaging which in 2021 accounted for 12% of worldwide solid waste. Of the commonly bulk-manufactured plastics, PET has the highest greenhouse gas (GHG) emissions associated with its production (up to 4.5 kg GHG/kg PET) yet is one of the most amenable to various 'recycling' methodologies - which can be broadly be categorised as either incineration, mechanical- or chemical recycling. Incineration solves the landfill problem and can recover energy from the materials but has been determined after a recent study by the European Commission's Joint Research Centre to be less preferable from a climate change impact perspective. Mechanical recycling is operationally more facile yet amplifies existing degradation and produces recycled PET of lower value and quality, while chemical recycling *via* depolymerisation requires efficient process chemistry on large scales (frequently at high temperatures), however it can produce pure monomers which can be repolymerised to pristine PET for any application - thereby reducing reliance on fossil fuel-derived virgin PET. In addition, in the case of PET, a recent analysis found that CO₂ emission savings at end of life treatment are considerably lower for chemical recycling than for mechanical. Chemical recycling methodologies include (*inter alia*) aminolysis, alcoholysis, glycolysis and hydrolysis.

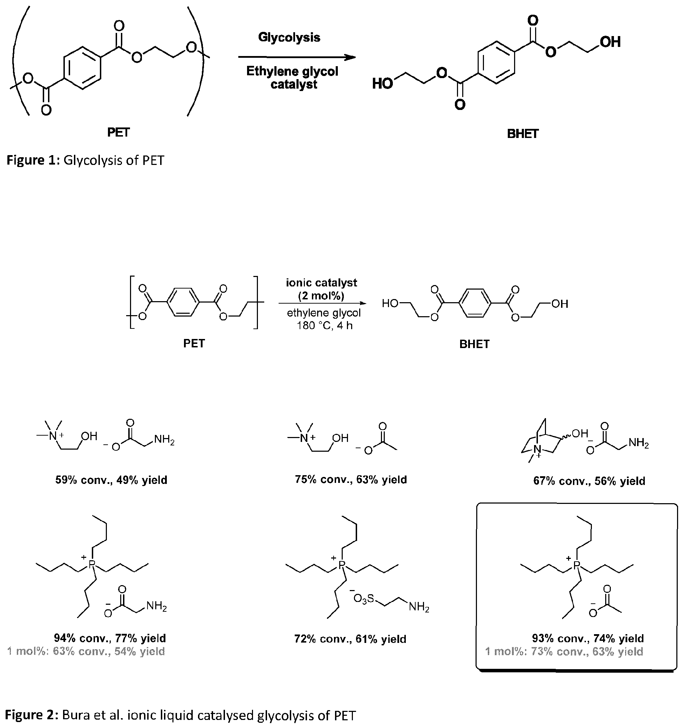

PET depolymerisation by glycolysis involves heating PET and ethylene glycol in the presence of a catalyst (see Figure 1). This results in the breakdown of the PET to form bis(2-hydroxyethyl) terephthalate monomer (BHET), which can be purified and polymerized to form new, virgin PET.

An array of species have emerged as suitable catalysts for glycolysis of PET, including metal salts, metal complexes, heterogenous materials, nanocatalysts, organocatalysts, deep eutectic solvents and ionic liquids (ILs).

Ghaemy et al. *(Polymer Degradation and Stability* **2005,** 90, 570) describe the depolymerisation of PET fibre using ethylene glycol in the presence of metal acetate as transesterification catalyst. The glycolysis reactions were carried out at the boiling point of ethylene glycol under nitrogen atmosphere for up to 10 hours. The effect of metal acetates as catalysts and their concentrations on yield was assessed. Four metal acetates were tested as catalysts, namely zinc acetate, cobalt acetate, manganese acetate and lead acetate. The order of catalytic activity was Zn²⁺>Mn²⁺>Co²⁺>Pb²⁺. Zinc acetate at a concentration of 1 wt% proved the most effective catalyst. Under the optimal conditions a monomer BHET yield of about 75% was achieved.

EP3377569B1 describes a method of making bis(2-hydroxyethyl) terephthalate (BHET) from polyethylene terephthalate (PET), comprising:
(a) combining PET with ethylene glycol and a catalyst system to produce a heterogeneous reaction mixture, wherein the catalytic system comprises a catalyst and a microwave absorber, with the microwave absorber being selected from the group consisting of a sodium salt, lithium salt, potassium salt, calcium salt, a magnesium salt, or combination thereof; and then
(b) heating by microwave irradiation the reaction mixture to produce a reaction product comprising BHET.

The following catalysts were assessed in the method: zinc acetate, zinc chloride, manganese acetate, 1,5,7 -triazabicyclo [4.4. 0] dec-5-ene (TBD), 1,8-diazabicyclo [5.4.O]undec-7-ene (DBU), magnesium acetate, and 4-dimethylaminopyridine (DMAP).

The preferred microwave absorber is sodium chloride. The preferred catalyst is zinc acetate.

WO2022004359A1 describes a method for producing BHET comprising depolymerizing polyester waste colored by a pigment, with a monoethylene glycol and a depolymerization catalyst, and depolymerizing the polyester waste to obtain a depolymerized product containing a crude colored BHET; a pigment decomposition/removal step for adding a pigment decomposition agent to the depolymerized product and cooling and centrifuging the resultant mixture, thereby separating extracted solids and the decomposed product of the pigment and removing at least a portion of the decomposed product from the depolymerized product; a concentration step for removing a low boiling-point component containing monoethylene glycol from the depolymerized product from which at least a portion of the decomposed product has been removed, and concentrating the crude BHET; and a collection step for collecting, by distillation, BHET from the concentrated crude BHET to obtain a BHET having higher purity than the crude BHET. The examples utilise sodium hydroxide as the depolymerisation catalyst.

Bura et al. (RSC Sustainability, 2023, 1, 2197) describe cholinium-based ionic liquid catalysts for PET glycolysis. The inventors also demonstrated that the activity of the biodegradable catalyst cholinium acetate could be improved upon by simplifying both the anion (to acetate) and the cation to the relatively non-polar tetrabutylphosphonium moiety. Mechanistic (computational) studies indicated that hydrogen-bond stabilisation by ethylene glycol was involved in catalysis and thus H-donating functionality was unlikely to be a useful catalyst structural feature. The conditions utilised were: 1g PET, 3.6 mL ethylene glycol, 1-2 mol% catalyst and heating to 180 °C (see Figure 2).

Pedrini et al. (ACS Sus. Chem. Eng. 2025, 13, 1424) describe ionic liquid catalysts for PET glycolysis, and identified biodegradable quaternary ammonium carboxylates as suitable catalysts for PET glycolysis, with yields of BHET as high as 76% recorded. In this work, a wide variety of anions were assessed with tetrabutylphosphonium catalysts, which led to the identification of hydrocinnamate as the most effective anion. Subsequently, a variety of phosphonium and alkylammonium ions were assessed with hydrocinnamate counterions as catalysts, which ultimately led to the identification of a biodegradable hydrocinnamate bis alkylated pyrolidinium catalyst, a benzoate pyrolidinium catalyst, and a glyceryl tioleate tetralkl ammonium catalyst, which were effective in catalysing glycolysis of PET.

Mendiburu-Valor et al. (Polymers 2021, 13, 1461) disclose depolymerisation of PET by glycolysis into BHET monomer working under pressure at 3 bar and elevated temperature (180°C, 220°C) using zinc acetate as a depolymerisation catalyst.

Imran et al. (Polym. Degrad. Stab., 2010, 95, 1686) describe depolymerization of PET by a supercritical method using ethylene glycol at a temperature of 450°C and a pressure of 15.3 MPa. A subcritical method was also disclosed wherein glycolysis was carried out at 350°C and 2.49 MPa or at 300°C and 1.1 MPa. An accelerated reaction rate was observed under supercritical conditions compared to subcritical conditions. In the case of the supercritical process, a maximum yield of 93.5% BHET was achieved within 30 minutes. A disadvantage of the disclosed methods is that very high temperature and pressure are needed to carry out the depolymerisation reactions.

Carné Sánchez et al. (Eur. Polym. J. 2011, 47, 1970) analysed the glycolysis of PET using a variety of zinc catalysts, including zinc acetate, zinc stearate and zinc sulfate, with zinc acetate proving to be the most soluble and effective catalyst.

It would be desirable to provide efficient methods for glycolysis of polymers comprising polyethylene terephthalate groups, such as polyethylene terephthalate (PET). In particular, it would be desirable to provide efficient methods for glycolysis of polymers including PET units, such as PET, which is environmentally more efficient, and which can be effectively carried out with PET flakes which have not been ground to sub-millimetre particle size, preferably where the method is suitable for depolymerizing PET flakes of larger size e.g. circa 5 mm squares, which is consistent with output from mechanical PET recycling facilities, as well as for depolymerizing smaller sized PET particles, and PET textiles. It would be desirable to provide efficient methods for glycolysis of PET using non-toxic catalysts, and under reaction conditions that require lower energy inputs.

### SUMMARY

In one aspect, the present invention provides a method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a magnesium salt or calcium salt of a carboxylic acid having the formula: 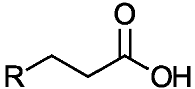 wherein R is selected from a C₆-C₂₀ aliphatic group, wherein R is optionally substituted with one or more: C₁-C₆ alkyl or C₁-C₆ alkoxy.

Suitably, R is a C₆-C₂₀ aliphatic group, optionally a C₆-C₂₀ alkyl group.

For example, R may be a C₆-C₁₈ aliphatic group, optionally, C₇-C₁₈ aliphatic group, such as C₇-C₁₇ aliphatic group.

Suitably, R is a C₆-C₁₈ alkyl group, optionally, C₇-C₁₈ alkyl group, such as C₇-C₁₇ alkyl group.

For example, R may be a C₇-C₁₇ alkyl chain, optionally a C₈-C₁₂ alkyl group.

The catalyst may be a solid in ethylene glycol at room temperature.

Suitably, the catalyst comprises a magnesium salt.

Additionally or alternatively, the catalyst may comprise a calcium salt.

The catalyst may comprise a magnesium salt of one or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a calcium salt of one or more of nonanoic acid (C₉), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably, the catalyst may comprise one or more of magnesium nonanoate, magnesium decanoate, magnesium undecanoate, magnesium dodecanoate, magnesium tridcanoate, magnesium tetradecanoate, magnesium pentadecanoate, magnesium hexadecanoate, magnesium margarate, magnesium stearate, magnesium nonadecanoate, magnesium arachidonate, magnesium heneicosanoate, and/or magnesium behenoate.

Suitably, the catalyst may comprise one or more of calcium nonanoate, calcium decanoate, calcium undecanoate, calcium dodecanoate, calcium tridcanoate, calcium tetradecanoate, calcium pentadecanoate, calcium hexadecanoate, calcium margarate, calcium stearate, calcium nonadecanoate, calcium arachidonate, calcium heneicosanoate, and/or calcium behenoate.

Optionally, the catalyst comprises catalyst comprises a magnesium salt or calcium salt of: nonanoic acid (C₉), decanoic acid (C₁₀), undecanoic acid (C₁₁), dodecanoic acid (C₁₂), tridecanoic acid (C₁₃), and/or tetradecanoic acid (C₁₄).

Further optionally, the catalyst comprises magnesium decanoate, magnesium undecanoate, magnesium dodecanoate, magnesium tridecanoate, magnesium tetradecanoate and/or magnesium stearate.

Suitably, the catalyst comprises magnesium decanoate, magnesium undecanoate, magnesium dodecanoate, magnesium tridecanoate, and/or magnesium tetradecanoate. More suitably, the catalyst comprises magnesium decanoate.

The catalyst may be employed in an amount of 5 mol% or less, suitably in an amount of 2 mol% or less, more suitably, in an amount of 1 mol% or less relative, most suitably in an amount of 0.5 mol% or less per mole of monomeric repeating unit of polyethylene terephthalate.

For example, the catalyst may be employed in an amount of 0.01 mol% to 5 mol% per mole of monomeric repeating unit of polyethylene terephthalate, such as from 0.05 mol% to 2 mol% per mole of monomeric repeating unit of polyethylene terephthalate, for example from 0.1 mol% to 1 mol% per mole of monomeric repeating unit of polyethylene terephthalate, optionally, in an amount of from 0.1 mol% to 0.5 mol% per mole of monomeric repeating unit of polyethylene terephthalate. Suitably, the catalyst may be employed in an amount of 0.01 mol% to 1 mol% per mole of monomeric repeating unit of polyethylene terephthalate.

Optionally, the catalyst is employed in an amount of from 0.01 wt% to 5 wt% based on the total weight of PET, for example, the catalyst may be employed in an amount of from 0.05 wt% to 2 wt% based on the total weight of PET, such as from 0.05 wt% to 1.5 wt% based on the total weight of PET, optionally, in an amount of from 0.1 wt% to 1 wt%, such as from 0.1 wt% to 0.5 wt% based on the total weight of PET.

The the weight ratio of PET:ethylene glycol may be in the range of from 1:1 to 1:20, suitably in the range of from 1:2 to 1:15, preferably in the range of from 1:3 to 1:12. Suitably, the PET:ethylene glycol is in the weight ratio of from 1:1 to 1:10, optionally from 1:3 to 1:6, such as 1:4.

Optionally, the catalyst may comprise a mixture of two or more magnesium salts of a carboxylic acid having the formula: 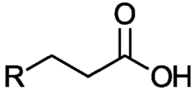 wherein R is selected from a C₆-C₂₀ aliphatic group, wherein R is optionally substituted with one or more: C₁-C₆ alkyl or C₁-C₆ alkoxy.

Optionally, the catalyst may comprise a mixture of two or more calcium salts of a carboxylic acid having the formula: 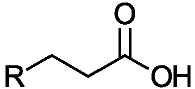 wherein R is selected from a C₆-C₂₀ aliphatic group, wherein R is optionally substituted with one or more: C₁-C₆ alkyl or C₁-C₆ alkoxy.

The catalyst may comprise calcium or magnesium salts of two or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a calcium salt of two or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a magnesium salt of two or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably, the catalyst may comprise two or more of magnesium nonanoate, magnesium decanoate, magnesium undecanoate, magnesium dodecanoate, magnesium tridcanoate, magnesium tetradecanoate, magnesium pentadecanoate, magnesium hexadecanoate, magnesium margarate, magnesium stearate, magnesium nonadecanoate, magnesium arachidonate, magnesium heneicosanoate, and/or magnesium behenoate.

Suitably, the catalyst may comprise two or more of calcium nonanoate, calcium decanoate, calcium undecanoate, calcium dodecanoate, calcium tridcanoate, calcium tetradecanoate, calcium pentadecanoate, calcium hexadecanoate, calcium margarate, calcium stearate, calcium nonadecanoate, calcium arachidonate, calcium heneicosanoate, and/or calcium behenoate.

Optionally, the catalyst may comprise two or more magnesium and/or calcium salts of: nonanoic acid (C₉), decanoic acid (C₁₀), undecanoic acid (C₁₁), dodecanoic acid (C₁₂), tridecanoic acid (C₁₃), and/or tetradecanoic acid (C₁₄).

The catalyst may comprise three or more or four or more of magnesium and/or calcium salts of: nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably the catalyst comprises magnesium decanoate, and one or more of magnesium undecanoate, magnesium dodecanoate, magnesium tridecanoate, magnesium tetradecanoate and/or magnesium stearate.

Suitably, the catalyst comprises magnesium decanoate and two or more of: magnesium salts of nonanoic acid, decanoic acid, myristic acid, palmitic acid, lauric acid, stearic acid, oleic acid, linoleic acid.

For example, the catalyst may comprise magnesium decanoate, magnesium palmate, and magnesium stearate. The catalyst may comprise magnesium decanoate, magnesium stearate and magnesium oleate. The catalyst may comprise magnesium decanoate magnesium oleate and magnesium linoleate.

Suitably the catalyst comprises calcium decanoate, and one or more of calcium undecanoate, calcium dodecanoate, calcium tridecanoate, calcium tetradecanoate and/or calcium stearate.

Suitably, the catalyst comprises calcium decanoate and two or more of: calcium salts of nonanoic acid, decanoic acid, myristic acid, palmitic acid, lauric acid, stearic acid, oleic acid, linoleic acid.

For example, the catalyst may comprise calcium decanoate, calcium palmate, and calcium stearate. The catalyst may comprise calcium decanoate, calcium stearate and calcium oleate. The catalyst may comprise calcium decanoate, calcium oleate and calcium linoleate.

Optionally, the method further comprises a filtration step to remove the catalyst from a mixture comprising the BHET.

In another aspect, the present invention provides a method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a mixture of magnesium carboxylate salts and/or calcium carboxylate salts formed from the hydrolysis of a plant oil or animal fat.

Suitably, the catalyst comprises one or more magnesium salts of: decanoic acid, palmitic acid, tetradecanoic acid, palmitic acid, stearic acid, oleic acid, linoleic acid and arachidonic acid.

Suitably, the catalyst comprises one or more calcium salts of: decanoic acid, palmitic acid, tetradecanoic acid, palmitic acid, stearic acid, oleic acid, linoleic acid and arachidonic acid.

Optionally, the plant oil is selected from vegetable oil, coconut oil, sunflower oil, peanut oil, rice oil, flaxseed oil, sesame oil, grape seed oil, rapeseed oil, olive oil, canola oil, and/or corn oil.

Optionally, the mixture of mixture of magnesium carboxylate salts formed from the hydrolysis of a plant oil or animal fat includes a mixture of two or more magnesium salts of decanoic acid, dodecanoic acid, tetradecanoic acid, palmitic acid, stearic acid, oleic acid, linoleic acid and arachidonic acid.

For example, the mixture of magnesium carboxylate salts formed from the hydrolysis of a plant oil or animal fat may comprise a mixture of magnesium salts having the formula: 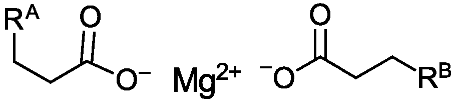 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

For example, the mixture of calcium carboxylate salts formed from the hydrolysis of a plant oil or animal fat may comprise a mixture of calcium salts having the formula: 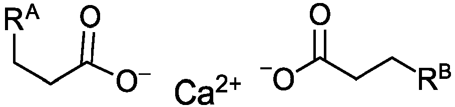 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

Suitably, the plant oil or animal fat comprises waste cooking oil.

Optionally, the catalyst is present in an amount of from 0.05 wt% to 5 wt% based on the weight of the polyethylene terephthalate, optionally from 0.1 wt% to 4 wt% based on the weight of the polyethylene terephthalate, further optionally from 0.1 to 1 wt% based on the weight of the polyethylene terephthalate.

### BRIEF DESCRIPTION OF THE DRAWINGS

**Fig. *1*** is a scheme for glycolysis of PET.
**Fig. *2*** shows the work of Bura et al. (RSC Sustainability, 2023, 1, 2197)
**Fig. 3** is a scheme for the formation of magnesium carboxylates from triglycerides which may be present or derived from plant oil or animal fat.
**Fig. *4*** is scheme showing various magnesium salts that may be formed from a mixture two different types of carboxylate anion.

### DETAILED DESCRIPTION

As outlined above, the present invention provides a method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a magnesium salt or calcium salt of a carboxylic acid having the formula: 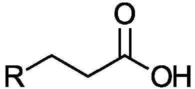 wherein R is selected from a C₆-C₂₀ aliphatic group, wherein R is optionally substituted with one or more: C₁-C₆ alkyl or C₁-C₆ alkoxy.

The method involves glycolysis of a polymer comprising PET units, such as PET, using calcium or magnesium carboxylate salts.

R is a C₆-C₂₀ aliphatic group, wherein R is optionally substituted with one or more: C₁-C₆ alkyl or C₁-C₆ alkoxy. When R may contain one or more or no unsaturated bonds. For example, R may contain a single double bond, or two double bonds, or three double bonds. Additionally, or alternatively, R may contain a single triple bond, or two triple bonds, or three triple bonds.

Suitably, R comprises one or two or no double bonds.

R may be a C₆ aliphatic group, a C₇ aliphatic group, C₈ aliphatic group, C₉ aliphatic group, C₁₀ aliphatic group, C₁₁ aliphatic group, C₁₂ aliphatic group, C₁₃ aliphatic group, C₁₄ aliphatic group, C₁₅ aliphatic group, C₁₆ aliphatic group, C₁₇ aliphatic group, C₁₈ aliphatic group, C₁₉ aliphatic group, C₂₀ aliphatic group, or a C₂₀ aliphatic group.

For example, R may be a C₆-C₁₈ aliphatic group, optionally, C₇-C₁₈ aliphatic group, such as C₇-C₁₇ aliphatic group.

R may be a C₆-C₂₀ alkyl group, optionally substituted with one or more: C₁-C₆ alkyl or C₁-C₆ alkoxy.

R may be a C₆ alkyl group, a C₇ alkyl group, C₈ alkyl group, C₉ alkyl group, C₁₀ alkyl group, C₁₁ alkyl group, C₁₂ alkyl group, C₁₃ alkyl group, C₁₄ alkyl group, C₁₅ alkyl group, C₁₆ alkyl group, C₁₇ alkyl group, C₁₈ alkyl group, C₁₉ alkyl group, C₂₀ alkyl group, or a C₂₀ alkyl group.

Suitably, R is a C₆-C₁₈ alkyl group, optionally, C₇-C₁₈ alkyl group, such as C₇-C₁₇ alkyl group.

For example, R may be a C₇-C₁₇ alkyl chain, optionally a C₈-C₁₂ alkyl group.

The catalyst may for example be a calcium or magnesium salt of: a monounsaturated C₉-C₂₄ carboxylic acid, such as myristoleic acid, palmitoleic acid, sapienic acid, oleic acid, elaidic acid, vaccenic acid, gadoleic acid, eicosenoic acid, or nervonic acid.

The catalyst may for example be a calcium or magnesium salt of: a di-unsaturated C₉-C₂₄ carboxylic acid, such as linoleic acid, eicosadienoic acid, or docosedienoic acid.

The catalyst may for example be a calcium or magnesium salt of: a tri-unsaturated C₉-C₂₄ carboxylic acid, such as linolenic acid, pinolenic acid, elostearic acid, mead acid, dihomo-γ-linolenic acid or eicosatrienoic acid.

The catalyst may for example be a calcium or magnesium salt of: a tetra-unsaturated C₉-C₂₄ carboxylic acid, such as stearidonic acid, arachidonic acid, eicosatetraenoic acid or adrenic acid.

The catalyst may be a solid in ethylene glycol at room temperature.

Suitably, the catalyst comprises a magnesium salt.

Additionally or alternatively, the catalyst may comprise a calcium salt.

The catalyst may comprise a magnesium salt of one or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a calcium salt of one or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably, the catalyst may comprise one or more of magnesium nonanoate, magnesium decanoate, magnesium undecanoate, magnesium dodecanoate, magnesium tridcanoate, magnesium tetradecanoate, magnesium pentadecanoate, magnesium hexadecanoate, magnesium margarate, magnesium stearate, magnesium nonadecanoate, magnesium arachidonate, magnesium heneicosanoate, and/or magnesium behenoate.

Suitably, the catalyst may comprise one or more of calcium nonanoate, calcium decanoate, calcium undecanoate, calcium dodecanoate, calcium tridcanoate, calcium tetradecanoate, calcium pentadecanoate, calcium hexadecanoate, calcium margarate, calcium stearate, calcium nonadecanoate, calcium arachidonate, calcium heneicosanoate, and/or calcium behenoate.

Optionally, the catalyst comprises catalyst comprises a magnesium salt or calcium salt of: nonanoic acid (C₉), decanoic acid (C₁₀), undecanoic acid (C₁₁), dodecanoic acid (C₁₂), tridecanoic acid (C₁₃), and/or tetradecanoic acid (C₁₄).

Further optionally, the catalyst comprises magnesium decanoate, magnesium undecanoate, magnesium dodecanoate, magnesium tridecanoate, magnesium tetradecanoate and/or magnesium stearate.

Suitably, the catalyst comprises magnesium decanoate, magnesium undecanoate, magnesium dodecanoate, magnesium tridecanoate, and/or magnesium tetradecanoate. More suitably, the catalyst comprises magnesium decanoate.

The catalyst may be employed in an amount of 5 mol% or less, suitably in an amount of 2 mol% or less, more suitably, in an amount of 1 mol% or less relative, most suitably in an amount of 0.5 mol% or less per mole of monomeric repeating unit of polyethylene terephthalate.

For example, the catalyst may be employed in an amount of 0.01 mol% to 5 mol% per mole of monomeric repeating unit of polyethylene terephthalate, such as from 0.05 mol% to 2 mol% per mole of monomeric repeating unit of polyethylene terephthalate, for example from 0.1 mol% to 1 mol% per mole of monomeric repeating unit of polyethylene terephthalate, optionally, in an amount of from 0.1 mol% to 0.5 mol% per mole of monomeric repeating unit of polyethylene terephthalate. Suitably, the catalyst may be employed in an amount of 0.01 mol% to 1 mol% per mole of monomeric repeating unit of polyethylene terephthalate.

Optionally, the catalyst is employed in an amount of from 0.01 wt% to 5 wt% based on the total weight of PET, for example, the catalyst may be employed in an amount of from 0.05 wt% to 2 wt% based on the total weight of PET, such as from 0.05 wt% to 1.5 wt% based on the total weight of PET, optionally, in an amount of from 0.1 wt% to 1 wt%, such as from 0.1 wt% to 0.5 wt% based on the total weight of PET.

The the weight ratio of PET:ethylene glycol may be in the range of from 1:1 to 1:20, suitably in the range of from 1:2 to 1:15, preferably in the range of from 1:3 to 1:12. Suitably, the PET:ethylene glycol is in the weight ratio of from 1:1 to 1:10, optionally from 1:3 to 1:6, such as 1:4.

Optionally, the catalyst may comprise a mixture of two or more magnesium salts of a carboxylic acid having the formula: 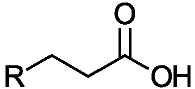 wherein R is selected from a C₆-C₂₀ aliphatic group, wherein R is optionally substituted with one or more: C₁-C₆ alkyl or C₁-C₆ alkoxy.

Optionally, the catalyst may comprise a mixture of two or more calcium salts of a carboxylic acid having the formula: 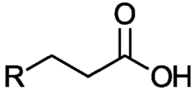 wherein R is selected from a C₆-C₂₀ aliphatic group, wherein R is optionally substituted with one or more: C₁-C₆ alkyl or C₁-C₆ alkoxy.

The catalyst may comprise calcium and/or magnesium salts of two or more of nonanoic acid (C₉), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a calcium salt of two or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

The catalyst may comprise a magnesium salt of two or more of nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably, the catalyst may comprise two or more of magnesium nonanoate, magnesium decanoate, magnesium undecanoate, magnesium dodecanoate, magnesium tridcanoate, magnesium tetradecanoate, magnesium pentadecanoate, magnesium hexadecanoate, magnesium margarate, magnesium stearate, magnesium nonadecanoate, magnesium arachidonate, magnesium heneicosanoate, and/or magnesium behenoate.

Suitably, the catalyst may comprise two or more of calcium nonanoate, calcium decanoate, calcium undecanoate, calcium dodecanoate, calcium tridcanoate, calcium tetradecanoate, calcium pentadecanoate, calcium hexadecanoate, calcium margarate, calcium stearate, calcium nonadecanoate, calcium arachidonate, calcium heneicosanoate, and/or calcium behenoate.

Optionally, the catalyst may comprise two or more magnesium or calcium salts of: nonanoic acid (C₉), decanoic acid (C₁₀), undecanoic acid (C₁₁), dodecanoic acid (C₁₂), tridecanoic acid (C₁₃), and/or tetradecanoic acid (C₁₄).

The catalyst may comprise three or more or four or more of magnesium and/or calcium salts of: nonanoic acid (C₉), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

Suitably the catalyst comprises magnesium decanoate, and one or more of magnesium undecanoate, magnesium dodecanoate, magnesium tridecanoate, magnesium tetradecanoate and/or magnesium stearate.

Suitably, the catalyst comprises magnesium decanoate and two or more of: magnesium salts of nonanoic acid, decanoic acid, lauric acid, myristic acid, palmitic acid, stearic acid, oleic acid, linoleic acid and linolenic acid.

Suitably, the catalyst comprises magnesium decanoate and two or more of: magnesium salts of nonanoic acid, decanoic acid, myristic acid, palmitic acid, lauric acid, stearic acid, oleic acid, linoleic acid.

For example, the two or more magnesium carboxylate salts a carboxylic acid having the formula: 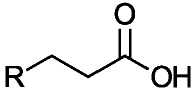 wherein R is selected from a C₆-C₂₀ aliphatic group, wherein R is optionally substituted with one or more: C₁-C₆ alkyl or C₁-C₆ alkoxy, may comprise a mixture of magnesium salts having the formula: 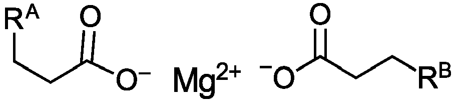 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

Optionally, the catalyst comprises calcium decanoate and two or more of: calcium salts of nonanoic acid, decanoic acid, myristic acid, palmitic acid, lauric acid, stearic acid, oleic acid, linoleic acid.

For example, the two or more calcium carboxylate salts a carboxylic acid having the formula: 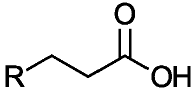 wherein R is selected from a C₆-C₂₀ aliphatic group, wherein R is optionally substituted with one or more: C₁-C₆ alkyl or C₁-C₆ alkoxy, may comprise a mixture of calcium salts having the formula: 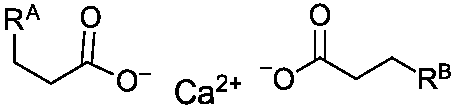 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

Optionally, the method further comprises a filtration step to remove the catalyst from a mixture comprising the BHET.

In another aspect, the present invention provides a method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a mixture of magnesium carboxylate salts and/or calcium carboxylate salts formed from the hydrolysis of a plant oil or animal fat.

Suitably, the catalyst comprises one or more magnesium and/or calcium salts of: decanoic acid, palmitic acid, tetradecanoic acid, palmitic acid, stearic acid, oleic acid, linoleic acid and arachidonic acid.

Optionally, the plant oil is selected from vegetable oil, coconut oil, sunflower oil, peanut oil, rice oil, flaxseed oil, sesame oil, grape seed oil, rapeseed oil, olive oil, canola oil, and/or corn oil.

Optionally, the mixture of mixture of magnesium carboxylate salts and/or calcium carboxylate salts formed from the hydrolysis of a plant oil or animal fat includes a mixture of two or more magnesium salts or calcium salts of carboxylic acids including two or more of decanoic acid, myristic acid, palmitic acid, margaric acid, stearic acid, oleic acid, linoleic acid, linoleic acid and arachidic acid.

Optionally, the mixture of magnesium carboxylate salts may comprise a magnesium salt including two different carboxylate anions.

Optionally, the mixture of calcium carboxylate salts may comprise a magnesium salt including two different carboxylate anions.

Optionally, the mixture of mixture of magnesium carboxylate salts formed from the hydrolysis of a plant oil or animal fat includes a mixture of two or more magnesium salts of decanoic acid, dodecanoic acid, tetradecanoic acid, palmitic acid, stearic acid, oleic acid, linoleic acid and arachidonic acid.

For example, the mixture of magnesium carboxylate salts formed from the hydrolysis of a plant oil or animal fat may comprise a mixture of magnesium salts having the formula: 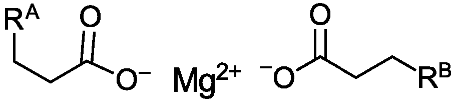 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

Optionally, the catalyst comprises one or more calcium salts of: decanoic acid, palmitic acid, tetradecanoic acid, palmitic acid, stearic acid, oleic acid, linoleic acid and arachidonic acid.

Optionally, the plant oil is selected from vegetable oil, coconut oil, sunflower oil, peanut oil, rice oil, flaxseed oil, sesame oil, grape seed oil, rapeseed oil, olive oil, canola oil, and/or corn oil.

Optionally, the mixture of calcium carboxylate salts formed from the hydrolysis of a plant oil or animal fat includes a mixture of two or more calcium salts of carboxylic acids including two or more of decanoic acid, myristic acid, palmitic acid, margaric acid, stearic acid, oleic acid, linoleic acid, linoleic acid and arachidic acid.

Optionally, the mixture of calcium carboxylate salts may comprise a calcium salt including two different carboxylate anions.

Optionally, the mixture of mixture of calcium carboxylate salts formed from the hydrolysis of a plant oil or animal fat includes a mixture of two or more calcium salts of decanoic acid, dodecanoic acid, tetradecanoic acid, palmitic acid, stearic acid, oleic acid, linoleic acid and arachidonic acid.

For example, the mixture of calcium carboxylate salts formed from the hydrolysis of a plant oil or animal fat may comprise a mixture of magnesium salts having the formula: 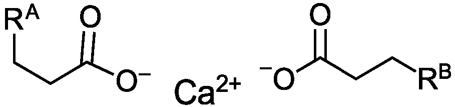 where R^{A} is a C₆-C₂₀ aliphatic group, and R^{B} is a C₆-C₂₀ aliphatic group.

Optionally, R^{A} is a C₆-C₂₀ alkyl group, and R^{B} is a C₆-C₂₀ aliphatic group.

Optionally, R^{B} is a C₆-C₂₀ alkyl group, and R^{A} is a C₆-C₂₀ aliphatic group.

Optionally, R^{A} is a C₆-C₂₀ alkyl group, and R^{B} is a C₆-C₂₀ alkyl group.

Optionally, at least one of R^{A} and R^{B} includes one or two or three double bonds.

Optionally, R^{A} and R^{B} are each a C₆-C₁₈ alkyl group, optionally, C₇-C₁₈ alkyl group, such as C₇-C₁₇ alkyl group.

For example, at least one of R may be a C₇-C₁₇ alkyl chain, optionally a C₈-C₁₂ alkyl group.

Suitably, at least one of R^{A} and R^{B} comprises a C₁₄, C₁₆, or C₁₈ alkyl group.

Optionally, R^{A} and R^{B} are not the same.

Suitably, the plant oil or animal fat comprises waste cooking oil.

Optionally, the catalyst is present in an amount of from 0.05 wt% to 5 wt% based on the weight of the polyethylene terephthalate, optionally from 0.1 wt% to 4 wt% based on the weight of the polyethylene terephthalate, further optionally from 0.1 to 1 wt% based on the weight of the polyethylene terephthalate.

Suitably, the plant oil or animal fat comprises waste cooking oil.

Optionally, the catalyst is present in an amount of from 0.05 wt% to 5 wt% based on the weight of the polyethylene terephthalate, optionally from 0.1 wt% to 4 wt% based on the weight of the polyethylene terephthalate, further optionally from 0.1 to 1 wt% based on the weight of the polyethylene terephthalate.

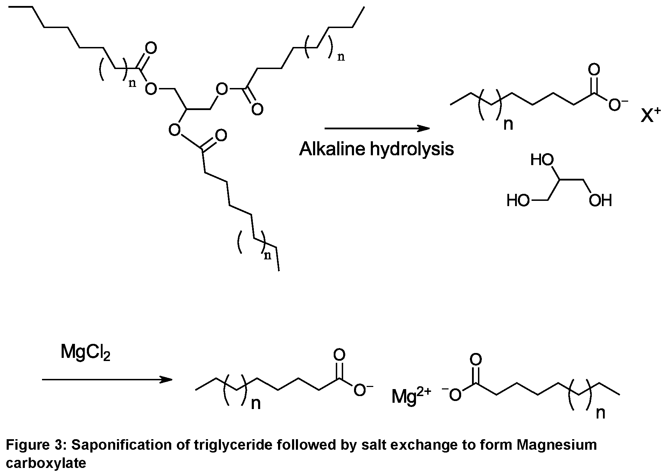

Figure 3 illustrates the alkaline hydrolysis of triglycerides, which are constituents of plant oils and animal fats. Treatment of a triglyceride with a hydroxide, such as sodium hydroxide or potassium hydroxide leads to the formation of carboxylate salts and glycerol. As a plant oil or animal fat comprises numerous triglycerides containing fatty acid chains of variable length, numerous carboxylate salts will form during the hydrolysis reaction, e.g. a mixture of C₁₀, C₁₂, C₁₄, C₁₆, C₁₈, C₂₀ carboxylate salts. The exact carboxylate content will be dependent on the fatty acid composition of the plant oil or animal fat being hydrolysed. Upon treatment of the carboxylate salts with a magnesium salt, such as magnesium chloride or magnesium bromide, the resulting magnesium carboxylates that form may contain two different anions, i.e. mixed carboxylate magnesium salts may form.

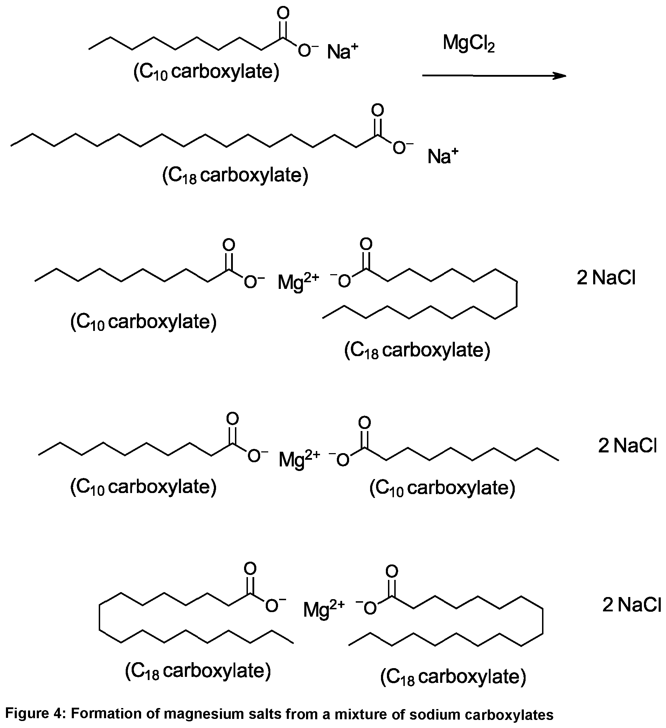

For example, a C₁₀ carboxylate anions and a C₁₈ carboxylate anion may coordinate with a magnesium cation to form three different magnesium salts: a C₁₀ magnesium salts, a C₁₈ carboxylate magnesium salts and a mixed carboxylate magnesium salt comprising a C₁₀ carboxylate and a C₁₈ carboxylate as shown in Figure 4.

Accordingly, in the method disclosed herein, the composition of a mixture of magnesium and/or calcium carboxylate salts formed from the hydrolysis of a plant oil or animal fat will depend on the fatty acid composition of the plant oil or animal fat from which the magnesium and/or calcium carboxylate salts are derived.

For example: the fatty acid content of coconut oil includes approx.: 5% Capric acid (C10), 56% lauric acid (C12), 22% myristic acid (C14), and 9% palmitic acid (C16).

Thus, a mixture of magnesium carboxylate salts formed from the hydrolysis of coconut oil, will comprise a mixture of magnesium salts comprising inter alia mixtures of C₁₀-C₁₆ carboxylates.

The fatty acid content of sunflower oil includes approx.: 28% C18:1 cis (n-9) (oleic acid) and approx. 62% C18:2cis (n6) (linoleic acid). Thus, a mixture of magnesium carboxylate salts formed from the hydrolysis of coconut oil, will comprise a mixture of magnesium salts comprising inter alia mixtures of C₁₈ carboxylates including oleic carboxylates and linoleic carboxylates.

The invention will be more readily appreciated by a review of the examples, which follow.

### EXAMPLES

The invention will be more readily appreciated by a review of the examples which follow.

To date, much of the investigations on PET depolymerization has employed smaller PET flakes, which have a larger surface area per gram of PET, and therefore represent more reactive, and less challenging substrates. In order to develop a platform for industrial application, we employed PET flakes of larger size, circa 5 mm square flakes, which is consistent with the output from mechanical PET recycling, unless indicated otherwise. In addition, we employed textile PET as an alternative substrate to assess substrate scope.

### Materials and Methods

The PET was derived from colourless water bottles purchased from a large supermarket chain. The average molecular weight for bottle grade PET ranges from 24000 to 36000 g mol⁻¹. The density of the PET was in the range of from 1.38 to 1.41 g/cm³.

Once emptied of their contents and labels were removed, the PET bottles were cut into flakes of approx. 5 mm square in size.

Proton Nuclear Magnetic Resonance (NMR) spectra were recorded on Bruker DPX 400 MHz and Bruker Avance II 600MHz spectrometers, using CDCl₃ and DMSO-*d*₆ as solvents, and referenced relative to residual CHCl₃ (δ = 7.26 ppm) or DMSO (δ = 2.50 ppm). Carbon NMR spectra were recorded on the same instruments (101 MHz and 151 MHz respectively) with total proton decoupling. Phosphorus NMR spectra were recorded on the Bruker DPX400 machine (162 MHz). Infrared spectra were obtained on a Perkin Elmer Spectrum 100 FT-IR spectrometer equipped with a universal ATR sampling accessory. ESI mass spectra were acquired using a Waters Micromass LCT- time of flight mass spectrometer (TOF), interfaced to a Waters 2690 HPLC. The instrument was operated in either positive or negative mode as required. APCI experiments were carried out on a Bruker microTOF-Q III spectrometer interfaced to a Dionex UltiMate 3000 LC or direct insertion probe. Agilent tuning mix APCI-TOF was used to calibrate the system. Hydrolysis experiments were carried out with Radleys Carousel 12 Plus Reaction Station. HPLC grade water was purchased from Sigma-Aldrich and used as the reaction medium for the basic hydrolysis of PET. Colourless polyethylene terephthalate bottles were purchased from a local large supermarket chain and cut into flakes (ca. 5 mm square). Unless otherwise noted, all commercially available compounds were used as provided, without any further purification.

### Catalyst Screening

As outlined above, the inventors assessed the efficacy of a wide variety of ionic catalysts in the glycolysis of polymers comprising PET units, such as PET.

Tetrabutyl phosphonium salts were studied to assess the influence of the cation on the glycolysis of PET (see Scheme 1 below): 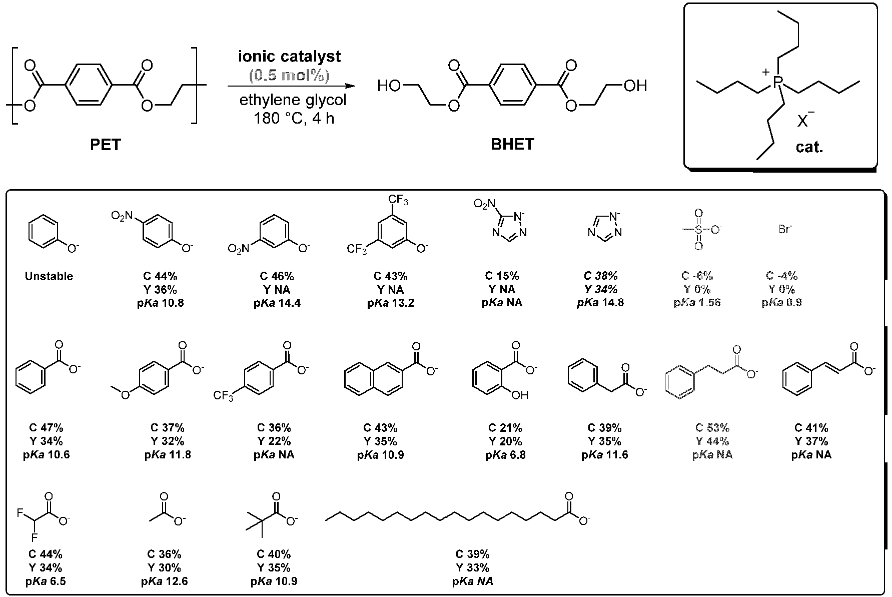 pKa = pKa of the corresponding conjugate acid in DMSO, Y = yield, C= Conversion.

Carboxylates outperformed other cations, and hydrocinnamate proved the most effective counter anion for tetrabutylphosphonium catalysed PET glycolysis under the conditions tested. Without being bound by theory, it was posited that π-stacking between the hydrocinnamate and PET occurs during the glycolysis reaction. This is in keeping with a late general base catalysis transition state where bond formation has progressed a significant extent and the effective p*K*ₐ of the attacking ethylene glycol nucleophile has lowered considerably relative to ethylene glycol itself.

The inventors then retained the hydrocinnamate anion and systematically varied the catalyst cation (see Scheme 2). 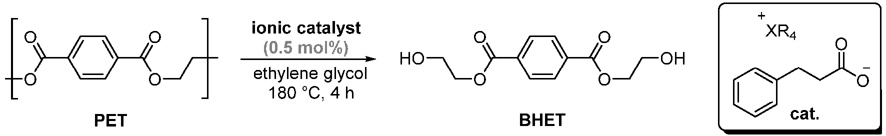 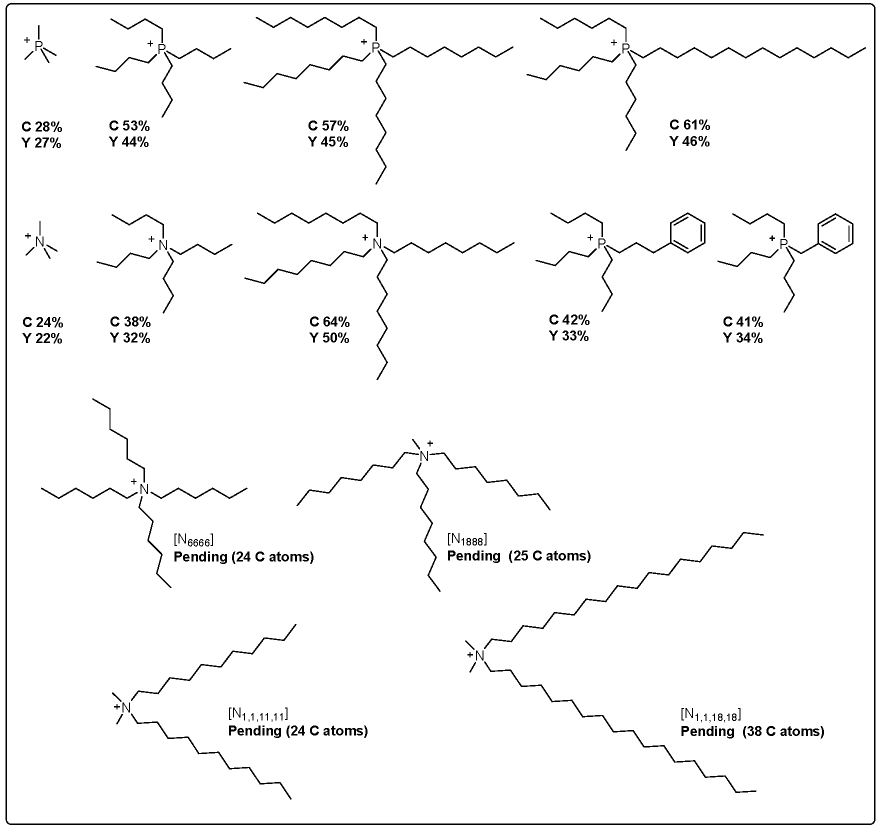 C = Conversion, Y = Yield.

While increasing the lipophilicity of the cation led to increased yield, incorporation of aromatic units proved less beneficial.

Given the contribution of the cation to catalyst activity, and the known efficacy of metal acetates in PET glycolysis, the inventors examined the use of metal carboxylate salts as promoters of depolymerisation of PET flakes.

As outlined above, metal acetates have demonstrated some efficacy in PET depolymerisation. To date, research efforts have focused on heavy M²⁺ ions bound to acetate, such as Zn(OAc)₂, Co(OAc)₂, Pb(OAc)₂, Mn(OAc)₂ and Cu(OAc)₂ (see Wong et al. J. Polym. Sci. A. 1989, 27, 2071).

The inventors screened a variety of metal carboxylates for PET depolymerisation, employing 5mm x 5mm PET flakes, a catalyst loading of 0.5 mol% per mole of PET units and with a reaction time of 2 hours (see Table 1).

| **Table 1: Performance of various metal carboxylate salts in the glycolysis of PET.** | | | | | |
|---|---|---|---|---|---|
| **Catalyst** | **PET** | **Catalyst Loading** | **Conversion (%)** | **Yield (%)** | **Reaction duration** |
| NaOAc | Flakes | 0.5 | 30 | 25 | 2h |
| LiOAc | Flakes | 0.5 | 37 | 29 | 2h |
| Zn(OAc)₂ | Flakes | 0.5 | 88 | 61 | 2h |
| Mg(OAc)₂ | Flakes | 0.5 | 89 | 70 | 2h |
| Mg(hydrocinnamate)₂ | Flakes | 0.5 | 90 | 75 | 2h |
| Mg(stearate)₂ | Flakes | 0.5 | 90 | 74 | 2h |
| Ca(OAc)₂ | Flakes | 0.5 | 91 | 67 | 2h |
| Ba(OAc)₂ | Flakes | 0.5 | 76 | 60 | 2h |
| Sr(OAc)₂ | flakes | 0.5 | 76 | 58 | 2h |

Surprisingly magnesium and calcium carboxylates proved particularly useful as catalysts in PET glycolysis. In particular, while Zn(OAc)₂ is known in the art to be an effective catalyst for PET glycolysis, other zinc carboxylates have proved significantly less effective. Consequently, it was surprising that the long chain magnesium stearate catalyst outperformed both magnesium acetate and zinc acetate under the test conditions.

To be useful for industrial application, it is important that a wide variety of sources/forms of PET would be suitable substrates for the glycolysis reaction. To be certain that the magnesium stearate catalyst was superior to ionic-liquid variants tested, regardless of the source of the PET, the inventors compared the performance of magnesium stearate with [N₈₈₈₈][hydrocinnamate] in the depolymerisation of PET flakes from commercial beverage bottles for 2h and PET granules of 3-5 mm size for 4 h. The results are shown below in Table 2. Magnesium stearate proved more efficacious catalyst regardless of the source/form of the PET.

| **Table 2: Influence of PET form/source on PET glycolysis** | | | | | |
|---|---|---|---|---|---|
| **Catalyst** | **PET** | **Catalyst Loading** | **Conversion (%)** | **Yield (%)** | **Reaction duration** |
| [N₈₈₈][hydrocinnamate] | Granules | 0.5 | 50 | 32 | 4 h |
| [N₈₈₈][hydrocinnamate] | Flakes | 0.5 | 59 | 43 | 2 h |
| Mg(stearate)₂ | Granules | 0.5 | 81 | 67 | 4 h |
| Mg(stearate)₂ | Flakes | 0.5 | 90 | 74 | 2 h |

The influence of ethylene glycol levels on BHET yield was subsequently assessed with Mg(stearate)₂ as the catalyst. Reactions were run using 1:4 PET: ethylene glycol (w/w) and 1:10 PET:ethylene glycol (w/w). The yield of the process under standard conditions (catalyst = Mg(stearate)₂ , catalyst loading = 0.5 mol%, PET source = PET flakes 5mm x 5mm, reaction time = 2 hours) is 88% (1:4 PET:ethylene glycol), which rises to 95% when more ethylene glycol is utilised (1:10 PET:ethylene glycol).

Next, the effect of alkyl chain length of the carboxylate anion on catalyst efficacy was assessed.

| **Table *4*** Effect of alkyl chain length on catalysis and their comparison with benchmark catalysts (stirring speed - 800 rpm and PET flakes) | | | | | | | |
|---|---|---|---|---|---|---|---|
| **Catalyst** | **chain** | **PET:EG (*wt:wt*)** | **loading (mol%)** | **Conv. (%)** | **Yield (%)** | **NMR Yield (%)^{a}** | **time (h)** |
| Mg(Acetate)₂ | C2 | 1:4 | 0.5 | - | - | 52 | 1 |
| Mg(Hexanoate)₂ | C6 | 1:4 | 0.5 | - | - | 49 | 1 |
| Mg(Decanoate)₂ | C10 | 1:4 | 0.5 | - | - | 59 | 1 |
| Mg(Tetradecanoate)₂ | C14 | 1:4 | 0.5 | - | - | 57 | 1 |
| Mg(Stearate)₂ | C18 | 1:4 | 0.5 | - | - | 44 | 1 |
| Mg(Tetraeicosanoate)₂ | C24 | 1:4 | 0.5 | - | - | 23 | 1 |
| Mg(Oleate)₂ | C18 | 1:4 | 0.5 | - | - | 49 | 1 |
| Zn(Acetate)₂ | C2 | 1:4 | 0.5 | - | - | 39 | 1 |
| TBD:MSA | - | 1:4 | 0.5 | - | - | 2 | 1 |
| Urea | - | 1:4 | 0.5 | - | - | 1 | 1 |
| [P₈₈₈₈][Hydrocinnamate] | - | 1:4 | 0.5 | - | - | 33 | 1 |
| Mg(Decanoate)₂ | C10 | 1:10 | 1 | 95 | 77 | - | 2 |
| Mg(stearate)₂ | C18 | 1:4 | 0.5 | 90 | 74 | - | 2 |
| Mg(OAc)₂ | C2 | 1:4 | 0.5 | 89 | 70 | - | 2 |

Under the test conditions, magnesium decanoate (C₁₀) and magnesium tetradecanoate (C₁₄) proved to be the most effective catalysts, with yields of 59% and 57% observed. Interestingly, when a reaction time of 1 hour was employed, Mg(OAc)₂ slightly outperformed Mg(stearate), however, when the reaction time was extended to 2 hours, Mg(stearate)₂ surprisingly proved more effective.

Given the reported drop off in catalytic efficacy for longer chain Zinc carboxylates as catalysts of glycolysis, for example with Zn(stearate)₂ being less effective than Zn(OAc)2 as a catalyst for glycolysis of PET, it is particularly surprising that longer chain carboxylate magnesium salts proved more effective than Mg(OAc)₂. The inventors assessed if there is a fall off in activity with increasing chain length, and a carboxylates chain length of C₂₄ or higher resulted in a significant reduction in yield. As such, the inventors have identified a specific compound class, namely C₉-C₂₃ magnesium carboxylates that is surprisingly effective in catalysing the glycolysis of PET, with C₉-C₁₈ magnesium carboxylates proving particularly effective, and C₉-C₁₅ magnesium carboxylates proving to be the most effective catalysts of PET glycolysis. Such catalysts are non-toxic, inexpensive, can be readily synthesised, and the reaction conditions employed are less energy intensive than other prior art reaction conditions, which oft require prolonged reaction periods, higher temperatures, higher pressures, or microwave heating.

To assess whether the environmental impact of the PET glycolysis disclosed herein could be further enhanced, the inventors posited that an effective catalyst could be generated by saponification of long chain glycerides, and converting the resulting carboxylates to form magnesium carboxylates as disclosed herein.

A screen of suitable starting materials for generating mixtures of carboxylates which can then be converted to the corresponding magnesium salts was carried out. The inventors hypothesised that plant oils and animal fats could be hydrolysed to generate mixtures of carboxylates including C₉-C₂₃ aliphatic carboxylates from which magnesium salts could be generated which in turn could be employed to catalyse PET glycolysis.

The inventors hydrolysed each of: vegetable oil, coconut oil, restaurant cooking oil, waste restaurant cooking oil, and sunflower oil. The oils/fats in question were hydrolysed using sodium hydroxide at elevated temperature to form a mixture of sodium carboxylate salts, which were then treated converted to magnesium salts by treating with magnesium chloride. The magnesium salts were isolated by filtration and dried overnight at 60°C in a vacuum oven.

Table 5 provides the results of optimisation studies using mixtures of magnesium carboxylates prepared from vegetable oils and/or animal fats.

| **Sr. No.** | **Catalyst (w/w%)** | **Time (h)** | **Temp. (°C)** | **NMR yield (%)** | **Isolated Yield (%)** |
|---|---|---|---|---|---|
| **1** | Mg (stearate) 0.5 mol% | 2 | 180 | | 82.3 |
| **2** | Mg (stearate) 0.5 mol% | 1 | 180 | 42.7 | - |
| **3** | RWO (3.5) | 2 | 190 | - | - |
| **4** | RWO (3.5) | 1.5 | 190 | 92.5 | - |
| **5** | RWO (3.5) | 45 min | 190 | 89 | - |
| **6** | RWO (1.5) | 1 | 190 | 91 | - |
| **7** | RWO (1.5) | 50 min | 190 | 92.4 | - |
| **8** | RWO (1.5) | 30 min | 190 | 54.8 | - |
| **9** | SO (1.5) | 30 min | 190 | 67.7 | - |
| **10** | CO (1.5) | 30 min | 190 | 79.6 | - |
| **11** | Mg stearate (1.5) | 30 min | 190 | 35.8 | - |
| **12** | RWO (1.5) | 15+15 min | 190 | 58.5 | - |
| **13** | VO(1.5) | 30 min. | 190 | 72.4 | - |
| **14** | Mg C10 (1.5) | 30 min. | 190 | 94.2 | - |
| **15** | RFO (1.5) | 30 min | 190 | 66.4 | - |
| **16** | Mg C10 (0.5) | 30 min. | 190 | 52.2 | - |
| **17** | Mg C10 (0.1) | 30 min. | 190 | 15 | - |
| **18** | Mg C10 (0.5) | 1 | 190 | 92.4 | 84.1 |
| **19** | Mg C10 (0.3) | 1 | 190 | 91 | 83.6 |
| **20** | CO (0.3) | 1 | 190 | 78.5 | - |
| **21** | CO (0.3) | 70 min | 190 | 91.2 | - |
| **22** | CO (0.4) | 1 | 190 | 89.4 | 80.8 |
| **23** | RWO (1.5) | 50 min | 190 | - | 80.7^{a} |
| **24** | RWO (1.5) | 50 min | 190 | - | 83.4^{b} |
| **25** | RWO (1.5) | 50 min | 190 | - | 83.0^{c} |
| **26** | TR RWO (1.5) | 40 min | 190 | 88.4 | - |
| ^{a}Volume concentrated to 60 mL during workup. ^{b}Volume concentrated to 40 mL during workup. ^{c}Volume concentrated to 50 mL during workup | | | | | |
| RWO = Restaurant Waste Cooking Oil (Bitebox, Pearse St, 5 days use) | | | | | |
| RFO = Restaurant Fresh Cookig Oil (same restaurant, unused) | | | | | |
| VO = Vegetable oil (Dunnes Stores, unused) | | | | | |
| CO = Coconut oil (pure, Dunnes Stores, unused) | | | | | |
| SO = Sunflower oil (pure, Dunnes Stores, unused) | | | | | |
| TR = Textile Reaction (PET source is textile waste not a bottle) | | | | | |
| w/w% = weight% (mole% impossible as catalyst is not characterised) | | | | | |
| Reaction conditions: 800 rpm stirring, EG = 3.6 ml, PET = 1.0 g | | | | | |

Pleasingly, each of the mixtures of magnesium carboxylates derived from the plant/animal oils catalysed the glycolysis of PET. Catalysts derived from coconut oil outperformed those derived from sunflower oil. Without being bound by theory, it is thought that this may be due to coconut comprising higher concentrations of C₁₀-C₁₈ fatty acids than sunflower oil. In addition, catalysts comprising saturated chains returned higher yields than catalysts comprising unsaturated chains. Advantageously, the inventors demonstrated that waste restaurant cooking oil, could be employed successfully to manufacture magnesium carboxylates that have shown excellent ability as catalysts in the glycolysis of PET. Thus the present methodology affords the opportunity to convert waste products into effective catalysts which can further be used to depolymerise PET.

The catalysts derived from waste oil were employed in up to 3.5 wt% based on the total weight of the PET, but excellent yields were achieved when only 1.5 wt% of catalyst was employed (see entries 22 and 23).

### Examples

### PET:EG (1:4, wt:wt) experimental procedure

An oven-dried carousel flask was charged with polyethylene terephthalate flakes (1.0000 g ± 0.0050 g, 5 mm x 5 mm), ethylene glycol (3.6 mL, 4 g) and magnesium stearate (0.0154 g, 0.026 mmol, 0.5 mol%).^{a,b} The flask was placed under an atmosphere of nitrogen (balloon), and the reaction was stirred for 4 h at 180 °C. The flask was removed from the carousel and was left to cool for 5 min. The reaction mixture was then diluted with deionised water (400 mL), and the resulting aqueous mixture was vigorously stirred for 20 min. Utilising a Bucker funnel the mixture was filtered and the pre weighed filter paper was dried overnight in a vacuum oven at 60 °C to determine levels of unreacted PET residue and oligomers. The filtrate was concentrated in vacuo to a total of 50 mL and cooled to 4 °C overnight to enhance crystallisation. The resulting crystals were filtered and dried overnight in a vacuum oven at 60 °C to obtain BHET as a crystalline white solid (0.9805 g, 74%).
^{a}Glycolysis experiments were accomplished with Radleys Carousel 12 Plus Reaction Station.
^{b}Mol% of catalyst is related to the number of moles of monomeric units (5.2 mmol) present in 1 g of polymer.

### PET:EG (1:4, wt:wt) experimental procedure to determine the yield by ¹H NMR spectroscopy

An oven dried carousel flask was charged with polyethylene terephthalate flakes (1.0000 g ± 0.0050 g, 5 mm x 5 mm), ethylene glycol (3.6 mL, 4 g) and magnesium stearate (0.0154 g, 0.026 mmol, 0.5 mol%).^{a,b} The flask was placed under an atmosphere of nitrogen (balloon), and the reaction was stirred for 4 h at 180 °C. The flask was removed from the carousel and was left to cool for 5 min. The reaction mixture was transferred in a 50 mL beaker, the internal standard was added ((*E*)-stilbene, 2 mmol, 0.3605 g) and the mixture was diluted by adding 15 mL of DMSO-*d*₆. The cloudy suspension was vigorously stirred while keeping the plate temperature to 50 °C until a clear solution save a small amount of insoluble catalyst. A sample of the reaction mixture (~ 3 drops) was diluted to ca. 1 mL with DMSO-*d*₆. The obtained sample was analysed by qH{¹³C}NMR to determine BHET yield (89%).^{c,d}
^{a}Glycolysis experiments were accomplished with Radleys Carousel 12 Plus Reaction Station.
^{b}Mol% of catalyst is related to the number of moles of monomeric units (5.2 mmol) present in 1 g of polymer.
^{c}qH{¹³C}NMR, 400 MHz, D1 = 15 s, GARP-mediated ¹³C decoupling.²
^{d}Yield value was calculated by subtraction of the -CH₂- signal at 3.72 ppm (4H) with the resonance associated with the soluble oligomeric fraction 4.68 ppm (4H).³
Resonances relevant for yield calculation:

| | | | |
|---|---|---|---|
| A: | 3.72 ppm | BHET (4H) + BHET dimer (4H) | |
| B: | 4.68 ppm | BHET dimer (4H) | |
| C: | 7.38 ppm | (E)-stilbene (4H) | set reference integration to 1.00 |

$%yield = \frac{\left(int.A-int.B\right)}{theoretical mmol} \times \frac{C mmol}{int . C} \times 100 = \frac{\left(int.A-int.B\right)}{5.2 mmol} \times 1 mmol \times 100 = 89 %$

### PET:EG (1:10, wt:wt) experimental procedure

An oven dried carousel flask was charged with polyethylene terephthalate flakes (0.5000 g ± 0.0025 g, 5 mm x 5 mm), ethylene glycol (4.5 mL, 5 g) and magnesium stearate (0.0077 g, 0.013 mmol, 0.5 mol%).^{a,b} The flask was placed under an atmosphere of nitrogen (balloon), and the reaction was stirred for 4 h at 180 °C. The flask was removed from the carousel and was left to cool for 5 min. The reaction mixture was then diluted with deionised water (200 mL), and the resulting aqueous mixture was vigorously stirred for 20 min. Utilising a Bucker funnel the mixture was filtered and the pre weighed filter paper was dried overnight in a vacuum oven at 60 °C to determine levels of unreacted PET residue and oligomers. The filtrate was evaporated to a total of 30 mL and cooled to 4 °C overnight to crystallise bis(2-hydroxyethyl)terephthalate (BHET). The resulting crystals were filtered and dried overnight in a vacuum oven at 60 °C to obtain BHET as a crystalline white solid (0.5276 g, 80%).
The mother liquor from the crystallisation process was evaporated to remove water and ethylene glycol; qH NMR analysis was performed using (*E*)-stilbene as the internal standard (1 mmol, 0.1803 g) to reveal the presence of 11% yield of BHET remaining in the crystallisation filtrate.
^{a}Glycolysis experiments were accomplished with Radleys Carousel 12 Plus Reaction Station.
^{b}Mol% of catalyst is related to the number of moles of monomeric units (2.6 mmol) present in 0.5 g of polymer.

### PET:EG (1:10, wt:wt) experimental procedure to determine the yield by ¹H NMR spectroscopy

An oven dried carousel flask was charged with polyethylene terephthalate (PET) flakes (0.5000 g ± 0.0025 g, 5 mm x 5 mm), ethylene glycol (4.5 mL, 5 g) and magnesium stearate

(0.0077 g, 0.013 mmol, 0.5 mol%).^{a,b} The flask was placed under an atmosphere of nitrogen (balloon), and the reaction was stirred for 4 h at 180 °C. The flask was removed from the carousel and was left to cool for 5 min. The reaction mixture was transferred in a 50 mL beaker, the internal standard was added ((*E*)-stilbene, 1 mmol, 0.1803 g) and the mixture was diluted by adding 10 mL of DMSO-*d*₆. The cloudy suspension was vigorously stirred while keeping the plate temperature to 50 °C until a clear solution save a small amount of insoluble catalyst. A sample of the reaction mixture (~ 3 drops) was diluted to ca. 1 mL with DMSO-*d*₆. The obtained sample was analysed by qH{¹³C}NMR to determine BHET yield (95%).^{c,d}
^{a}Glycolysis experiments were accomplished with Radleys Carousel 12 Plus Reaction Station.
^{b}Mol% of catalyst is related to the number of moles of monomeric units (2.6 mmol) present in 0.5 g of polymer.
^{c}qH{¹³C}NMR, 400 MHz, D1 = 15 s, GARP-mediated ¹³C decoupling.²
^{d}Yield value was calculated by subtraction of the -CH₂- signal at 3.72 ppm (4H) with the resonance associated with the soluble oligomeric fraction 4.68 ppm (4H).³
Resonances relevant for yield calculation:

| | | | | |
|---|---|---|---|---|
| A | - | 3.72 ppm | BHET (4H) + BHET dimer (4H) | |
| B | - | 4.68 ppm | BHET dimer (4H) | |
| C | - | 7.38 ppm | (E)-stilbene (4H) - | set integration to 1.00 |

$\begin{matrix}%yield = \frac{\left(int.A-int.B\right)}{theoretical mmol} \times \frac{C mmol}{int . C} ∗ 100 \\ = \frac{\left(int.A-int.B\right)}{2.6 mmol} \times 1 mmol ∗ 100 = 95 %\end{matrix}$

### Catalyst recyclability: experimental procedure

A two-necked 25 mL round bottomed flask equipped with a thermometer was charged with polyethylene terephthalate flakes (1.0000 g ± 0.0050 g, 5 mm x 5 mm), ethylene glycol (9.0 mL) and magnesium stearate (0.0615 g, 0.104 mmol, 2 mol%).^{a} The flask was placed under an atmosphere of nitrogen (balloon), and the reaction was stirred for 4 h at 180 °C (a clear transparent solution was obtained). The reaction vessel was left to cool for 5 min. The reaction mixture was then diluted with deionised water (400 mL), and the resulting aqueous mixture was vigorously stirred and heated to 90 °C. Utilising a pre-heated filter the insoluble catalyst was filtered, recovered from the mixture (0.0382 g, 62 %) and dried overnight in a vacuum oven at 60 °C. The filtrate was cooled to room temperature and traces of insoluble oligomeric material were removed by filtration. The filtrate was concentrated in vacuo to a total volume of 50 mL and cooled to 4 °C overnight to crystallise *bis*(2-hydroxyethyl)terephthalate (BHET). The resulting crystals were filtered and dried overnight in a vacuum oven to obtain BHET as a crystalline white solid (0.9601 g, 73%).

To assess the recyclability of the catalyst, a second experiment was performed in a carousel tube on 50% scale. The carousel tube was charged with polyethylene terephthalate flakes (0.5000 g ± 0.0025 g, 5 mm x 5 mm), magnesium stearate (0.0308 g, 0.052 mmol, 2 mol%) and ethylene glycol (4.5 mL).^{b} The tube was placed under an atmosphere of nitrogen (balloon), and the reaction was stirred for 4 h at 180 °C.° The reaction mixture was dilute with water (200 mL) and worked up using the previously stated procedure outlined above. Crystallisation was performed in 30 mL of aqueous solution at a temperature of 4 °C overnight. BHET was isolated as a crystalline white solid (0.5441 g, 82%).^{d} M.p. 103.0-104.5 °C (lit.,¹ M.p. 103.1-107.4 °C).
Product spectroscopic data correlated well to that in the literature.²
¹H NMR (400 MHz, DMSO-*d*₆): δ_{H} = 8.12 (s, 4H), 4.95 (t, 2H, *J* 5.3 Hz), 4.32 (t, 4H, *J* 4.7 Hz), 3.72 (dt, 4H, *J* 5.3 Hz, *J* 4.7 Hz) ppm.
^{a}Mol% of catalyst is related to the number of moles of monomeric units (5.2 mmol) present in 1 g of polymer.
^{b}Glycolysis experiments were carried out with Radleys Carousel 12 Plus Reaction Station.
^{c}temperature was measured in an adjacent carousel flask filled with the equivalent amount of ethylene glycol and equipped with a thermometer.
^{d}From a duplicate experiment quantitative recovery of the catalyst was detected.

### Synthesis of magnesium stearate

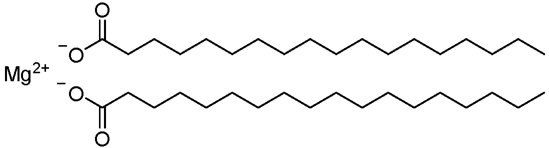

An oven-dried round-bottomed flask was charged with stearic acid (0.28 g, 1 mmol) and anhydrous Et2O (40 mL), the mixture was kept under argon atmosphere, it was stirred until complete dissolution of the acid and cooled to 0 °C. A solution of dibutylmagnesium (0.47 mL, 0.47 eq., 1 M in heptane) was added dropwise and formed a white precipitate. The resulting suspension was stirred for 1 h at room temperature and it was filtered with a Buckner funnel. The residue was washed with CHCl₃ (3 x 20 mL) and was dried in a vacuum oven for 4 h at 60° C to yield magnesium stearate as a white solid (0.21 g, 74%).
δ_{C} (101 MHz, CP/MAS SSNMR) 182.1, 38.0, 33.0, 27.4, 24.2, 14.4 ppm.
HRMS(m/z - DIP APCI⁻): Found: 283.2687 (M⁻) C₁₈H₃₅O₂⁻ Requires: 283.2643.
v_{MAX} (neat)/cm⁻¹: 2956, 2917, 2850, 1580, 1461, 1315, 1265, 1116, 885, 825, 767, 720.

### Synthesis of magnesium stearate

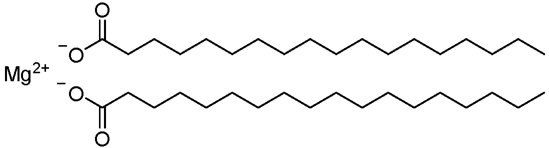

A 400 mL beaker was charged with deionised H₂O (300 mL) and stearic acid (1.42 g, 5 mmol), heated to 90 °C, and adjusted to pH 10. The suspension was stirred until full solubilisation of the acid was achieved. In a separate beaker, MgCl₂ (0.29 g, 3 mmol) was dissolved in H₂O (20 mL), and the solution was added dropwise to the first beaker at a rate of 1 mL per minute using a syringe pump to yield the precipitation of a white solid. The resulting suspension was gradually cooled to room temperature to favour flocculation of the precipitate. The solid was filtered, washed with H₂O (2 x 50 mL), and dried in a vacuum oven for 8 h at 60 °C to yield magnesium stearate as a white solid (1.46 g, 99%).
δ_{C} (101 MHz, CP/MAS SSNMR) 182.8, 182.4, 181.8, 180.6, 178.9, 40.6, 39.1, 37.5, 33.2, 28.3, 27.2, 26.6, 25.3, 24.3, 15.4, 14.7, 14.2 ppm.
HRMS (m/z - DIP APCI⁻): Found: 283.2675 (M⁻) C₁₈H₃₅O₂⁻ Requires: 283.2643.
v_{MAX} (neat)/cm-1: 2916, 2850, 1574, 1459, 1336, 1305, 1265, 1038, 877, 721, 687.

### Synthesis of magnesium hexanoate

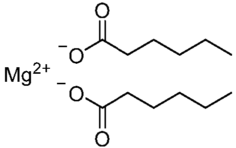

An oven-dried round-bottomed flask was charged with hexanoic acid (0.25 mL, 2 mmol) and anhydrous Et₂O (80 mL), the mixture was kept under argon atmosphere, it was stirred until complete dissolution of the acid and cooled to 0 °C. A solution of dibutylmagnesium (0.96 mL, 0.96 eq., 1 M in heptane) was added dropwise and formed a white precipitate. The resulting suspension was stirred for 1 h at room temperature and the solvent evaporated under reduced pressure. The residue was triturated with CHCl₃ (20 mL) and the resulting solid was filtered, washed with CHCl₃ (20 mL) and dried in a vacuum oven for 4 hat 60° C to yield **magnesium hexanoate** as a white solid (0.22 g, 88%). Dec.p. 332.6 °C (Measured at the onset of a TGA spectrum)
δ_{C} (101 MHz, CP/MAS SSNMR) 181.7, 37.8, 32.3, 26.0, 23.0, 14.1 ppm.
HRMS(m/z - DIP APCI⁻): Found: 115.0783 (M⁻) C₆H₁₁O₂⁻ Requires: 115.0765.
v_{MAX} (neat)/cm⁻¹: 2957, 2930, 2866, 1569, 1422, 1343, 1318, 1228, 1193, 1110, 963, 893, 850, 778, 731, 672.

### Synthesis of magnesium decanoate

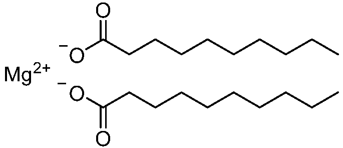

An oven-dried round-bottomed flask was charged with decanoic acid (0.35 g, 2 mmol) and anhydrous Et₂O (80 mL), the mixture was kept under argon atmosphere, it was stirred until complete dissolution of the acid and cooled to 0 °C. A solution of dibutylmagnesium (0.96 mL, 0.96 eq., 1 M in heptane) was added dropwise and formed a white precipitate. The resulting suspension was stirred for 1 h at room temperature and the solvent evaporated under reduced pressure. The residue was triturated with CHCl₃ (20 mL) and the resulting solid was filtered, washed with CHCl₃ (20 mL) and dried in a vacuum oven for 4 hat 60° C to yield **magnesium decanoate** as a white solid (0.34 g, 97%). Dec.p. 327.1 °C (Measured at the onset of a TGA spectrum)

### Synthesis of magnesium tetradecanoate

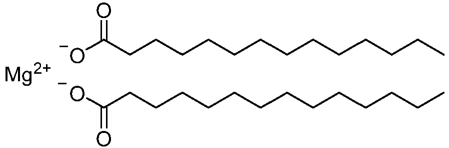

An oven-dried round-bottomed flask was charged with tetradecanoic acid (0.46 g, 2 mmol) and anhydrous Et₂O (40 mL), the mixture was kept under argon atmosphere, it was stirred until complete dissolution of the acid and cooled to 0 °C. A solution of dibutylmagnesium (0.96 mL, 0.96 eq., 1 M in heptane) was added dropwise and formed a white precipitate. The resulting suspension was stirred for 1 h at room temperature and the solvent evaporated under reduced pressure. The residue was triturated with CHCl₃ (20 mL) and the resulting solid was filtered, washed with CHCl₃ (20 mL) and dried in a vacuum oven for 4 hat 60° C to yield **magnesium tetradecanoate** as a white solid (0.30 g, 65%). Dec.p. 321.0 °C (Measured at the onset of a TGA spectrum)
δ_{C} (101 MHz, CP/MAS SSNMR) 181.4, 38.0, 34.3, 33.0, 27.3, 24.6, 14.3 ppm.
HRMS (m/z - DIP APCI⁻): Found: 227.2070 (M⁻) C₁₄H₂₇O₂⁻ Requires: 227.2017.
v_{MAX} (neat)/cm⁻¹: 2957, 2918, 2850, 1574, 1454, 1318, 1244, 1115, 721.

### Synthesis of magnesium tetracosanoate

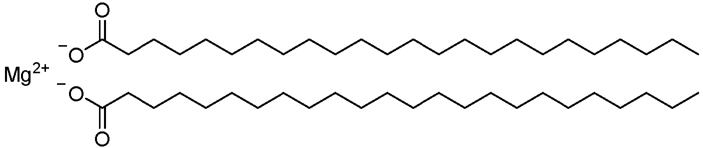

An oven-dried round-bottomed flask was charged with tetracosanoic acid (0.18 g, 0.49 mmol) and anhydrous Et₂O (20 mL), the mixture was kept under argon atmosphere, it was stirred until complete dissolution of the acid and cooled to 0 °C. A solution of dibutylmagnesium (0.47 mL, 0.47 eq., 1 M in heptane) was added dropwise and formed a white precipitate. The resulting suspension was stirred for 1 h at room temperature and it was filtered with a Buckner funnel. The residue was washed with CHCl₃ (3 x 10 mL) and was dried in a vacuum oven for 4 h at 60° C to yield **magnesium tetracosanoate** as a white solid (0.17 g, 88%).
δ_{C} (101 MHz, CP/MAS SSNMR) 182.2, 37.8, 36.1, 33.1, 27.3, 24.5, 14.4 ppm.
HRMS(m/z - DIP APCI⁻): Found: 367.3640 (M⁻) C₂₄H₄₇O₂⁻ Requires: 367.3582.
v_{MAX} (neat)/cm⁻¹: 2916, 2850, 1576, 1466, 1319, 1114, 719.

### Synthesis of magnesium oleate

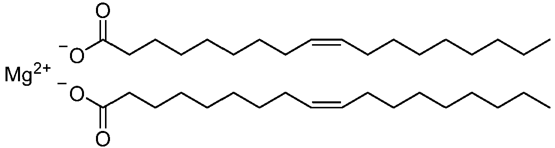

A 200 mL beaker was charged with deionised H₂O (100 mL) and oleic acid (0.32 mL, 1 mmol), heated to 90 °C, and adjusted to pH 10. The suspension was stirred until full solubilisation of the acid was achieved. In a separate beaker, MgCl₂ (0.07 g, 0.75 mmol) was dissolved in H₂O (10 mL), and the solution was added dropwise to the first beaker at a rate of 1 mL per minute using a syringe pump to yield the precipitation of a white solid. The resulting suspension was gradually cooled to room temperature to favour flocculation of the precipitate. The solid was filtered, washed with H₂O (2 x 25 mL), and dried in a vacuum oven for 8 h at 60 °C to yield **magnesium oleate** as a white solid (0.29 g, 99%).
δ_{C} (101 MHz, CP/MAS SSNMR) 182.7, 129.9, 37.7, 32.4, 30.5, 29.1, 28.6, 27.9, 26.7, 23.1, 14.3 ppm.
HRMS (m/z - DIPAPCl⁻): Found: 281.2540 (M⁻) C₁₈H3₃O₂⁻ Requires: 281.2486.
v_{MAX} (neat)/cm⁻¹: 2922, 2853, 1575, 1441, 1314, 721.

### Synthesis of oil-derived magnesium carboxylate catalysts

In a 50 mL flask, 5 g of oil, 2 g of NaOH, and 10 mL of water were added. The mixture was stirred at 90 °C for 3 h, during which the oil completely hydrolysed to the sodium carboxylates. The reaction mixture was then poured into a 500 mL beaker and diluted with 300 mL of distilled water. The temperature of the beaker was maintained at 90 °C. Once the solution reached this temperature, the pH was adjusted to ca. 9. Next, a solution of 2 g of MgCl₂ in 20 mL of water was added dropwise to the vigorously stirred hydrolysed oil solution. As the addition progressed, a solid precipitate began to form. After complete addition, the mixture was cooled to 0 °C, and the solid was filtered. Finally, the collected solid was dried in a vacuum oven at 60 °C overnight, yielding the oil-derived catalyst.

### Characterization of various oils:

**Vegetable oil (VO):**
¹³C NMR (101 MHz, solid state NMR) δ 184.9, 183.3, 182.2, 181.5, 180.8, 130.0, 128.3, 37.6, 32.4, 30.5, 27.8, 26.8, 23.1, 14.4.
v_{MAX}: 3007.78, 2922.68, 2853.16, 1745.67, 1574.36, 1449.03, 1238.62, 1162.14, 1049.75, 857.67, 721.02 cm⁻¹.

**Coconut oil (CO):**
¹³C NMR (101 MHz, solid state NMR) δ 182.8, 182.1, 180.9, 179.1, 130.0, 40.5, 39.2, 37.6, 36.4, 34.4, 33.0, 28.1, 26.7, 24.5, 14.4.
v_{MAX}: 2957.0, 2918.97, 2850.76, 1573.84, 1450.28, 1378.21, 1315.84, 1337.38, 1114.06, 1029.13, 933.22, 875.50, 789.50, 720.71 cm⁻¹.

**Restaurant fresh cooking oil (RFO):**
¹³C NMR (101 MHz, solid state NMR) δ 183.9, 182.8, 182.1, 180.6, 179.9, 130.0, 128.4, 38.0, 30.6, 27.9, 26.7, 23.1, 14.3.
v_{MAX}: 3010.35, 2921.80, 2852.43, 1567.13, 1442.41, 1315.93, 720.14 cm⁻¹.

**Restaurant waste cooking oil (RWO):**
¹³C NMR (101 MHz, solid state NMR) δ 185.1, 182.7, 182.0, 130.0, 128.4, 37.7, 33.0, 30.6, 27.8, 26.7, 23.1, 14.3.
v_{MAX}: 3009.83, 2921.58, 2852.26, 1573.45, 1446.06, 1311.83, 720.03 cm⁻¹.

**Sunflower oil (SO):**
¹³C NMR (101 MHz, solid state NMR) δ 184.7, 184.1, 182.0, 182.6, 130.0, 37.6, 32.4, 30.2, 23.1, 14.4.
v_{MAX}: 3053.37, 2923.16, 2853.31, 1743.54, 1576.65, 1437.29, 1315.46, 1059.59, 1003.73, 885.57, 722.10 cm⁻¹.

### Analysis of Different Oils via Comparison of Solid-State ¹³C NMR Spectra

| **Table 6: comparison of solid-state 13C NMR spectra of various oils** | | | | |
|---|---|---|---|---|
| **Oil Sample** | **Carbonyl Region (ppm)** | **Olefinic Region (ppm)** | **Aliphatic Region (ppm)** | **Key Observations** |
| **CO** | 182.8-179.1 | 130.00 | 40.5-14.4 | Higher saturation, shorter-chain fatty acids. |
| **VO** | 184.9-180.8 | 130.0, 128.3 | 37.6-14.4 | High unsaturation, long-chain triglycerides. |
| **RFO** | 183.9-179.9 | 130.0, 128.4 | 38.0-14.3 | Similar to VO, minimal degradation. |
| **RWO** | 185.1-182.0 | 130.0, 128.4 | 37.7-14.3 | Possible thermal degradation evident in upfield carbonyl shifts. |
| **SO** | 184.7-181.6 | 130.0 | 37.6-14.4 | High linoleic acid content, similar to VO |

**The NMR spectra reveal distinct compositional differences among the oils:**
- Coconut oil (CO) is more saturated with shorter fatty acid chains.
- Vegetable oil (VO) and sunflower oil (SO) have similar long-chain structures with high unsaturation.
- Restaurant waste oil (RWO) shows thermal degradation, evidenced by the carbonyl shift and reduced aliphatic signals.
- Restaurant fresh oil (RFO) retains a structure close to VO, confirming minimal degradation.

### General glycolysis procedure using oil/fat derived catalysts

An oven-dried carousel flask was charged with polyethylene terephthalate flakes (1.0 g, 5 mm x 5 mm), ethylene glycol (3.6 mL, 4 g) and oil-derived catalyst (w/w%).^{a} The flask was placed under an atmosphere of nitrogen (balloon), and the reaction was stirred for 50 min. at 190 °C. The flask was removed from the carousel and was left to cool for 5 min. The reaction mixture was then diluted with deionised water (400 mL), and the resulting aqueous mixture was vigorously stirred for 20 min. Utilising a Bucker funnel the mixture was filtered and the pre-weighed filter paper was dried overnight in a vacuum oven at 60 °C to determine levels of unreacted PET residue and oligomers. The filtrate was then concentrated under vacuum to a total volume of 50 mL and cooled to 4 °C overnight to promote crystallisation. The resulting crystals were filtered, the mother liquor further concentrated under vacuum to 25 mL, and cooled again to 4 °C overnight. The resulting crystals were filtered, combined with the first crop and dried overnight in a vacuum oven at 60 °C to obtain BHET as a crystalline white solid.
^{a}Glycolysis experiments were accomplished with Radleys Carousel 12 Plus Reaction Station.

### Glycolysis of PET using the waste oil-derived catalyst:

An oven-dried carousel flask was charged with polyethylene terephthalate flakes (1.0 g, 5 mm x 5 mm), ethylene glycol (3.6 mL, 4 g) and oil-derived catalyst (1.5 w/w%, 15 mg).^{a} The flask was placed under an atmosphere of nitrogen (balloon), and the reaction was stirred for 50 min. at 190 °C. The flask was removed from the carousel and was left to cool for 5 min. The reaction mixture was then diluted with deionised water (400 mL), and the resulting aqueous mixture was vigorously stirred for 20 min. Utilising a Bucker funnel the mixture was filtered and the pre-weighed filter paper was dried overnight in a vacuum oven at 60 °C to determine levels of unreacted PET residue and oligomers. The filtrate was then concentrated under vacuum to a total volume of 50 mL and cooled to 4 °C overnight to promote crystallisation. The resulting crystals were filtered, the mother liquor further concentrated under vacuum to 25 mL, and cooled again to 4 °C overnight. The resulting crystals were filtered, combined with the first crop and dried overnight in a vacuum oven at 60 °C to obtain BHET as a crystalline white solid (1.10 g, 83% yield).

### Glycolysis of textile waste using waste oil catalyst:

An oven-dried carousel flask was charged with textile cuttings (1.0 g, 5 mm x 5 mm), ethylene glycol (3.6 mL, 4 g) and oil-derived catalyst (1.5 w/w%, 15 mg).^{a} The flask was placed under an atmosphere of nitrogen (balloon), and the reaction was stirred for 50 min. at 190 °C. The flask was removed from the carousel and was left to cool for 5 min. The reaction mixture was then diluted with deionised water (400 mL), and the resulting aqueous mixture was vigorously stirred for 20 min. Utilising a Bucker funnel the mixture was filtered and the pre weighed filter paper was dried overnight in a vacuum oven at 60 °C to determine levels of unreacted textile residue and oligomers. The filtrate was then concentrated under vacuum to a total volume of 50 mL and cooled to 4 °C overnight to promote crystallization. The resulting crystals were filtered, mother liquor further concentrated under vacuum to 25 mL and cooled again to 4 °C overnight. The resulting crystals were filtered, combined with the first crop and dried overnight in a vacuum oven at 60 °C to obtain BHET as a crystalline white solid (1.06 g, 80% yield).

### Glycolysis of PET using waste oil catalyst at gram scale:

An oven-dried thick-walled glass tube was charged with polyethylene terephthalate flakes (5.0 g, 5 mm x 5 mm), ethylene glycol (18 mL, 20 g) and oil-derived catalyst (1.5 w/w%, 75 mg).^{a}The flask was placed under an atmosphere of nitrogen (balloon) with septum, and the reaction was stirred for 1 h at 190 °C. The thick-walled glass tube was removed from the oil bath and was left to cool for 5 min. The reaction mixture was then diluted with deionised water (2.0 L), and the resulting aqueous mixture was vigorously stirred for 20 min. Utilising a Bucker funnel the mixture was filtered and the pre weighed filter paper was dried overnight in a vacuum oven at 60 °C to determine levels of unreacted PET residue and oligomers. The filtrate was then concentrated under vacuum to a total volume of 300 mL and cooled to 4 °C overnight to promote crystallization. The resulting crystals were filtered, the mother liquor further concentrated under vacuum to 25 mL, and cooled again to 4 °C overnight. The resulting crystals were filtered, combined with the first crop and dried overnight in a vacuum oven at 60 °C to obtain BHET as a crystalline white solid (5.43 g, 82% yield).
¹H NMR (400 MHz, DMSO-*d*₆): δ_{H} = 8.12 (s, 4H), 4.95 (t, 2H, *J* 5.3 Hz), 4.32 (t, 4H, *J* 4.7 Hz), 3.72 (dt, 4H, *J* 5.3 Hz, *J* 4.7 Hz) ppm.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination.

### References

1 S. Javed, D. Ropel and D. Vogt, Green Chem., 2023, 25, 1442-1452.
2 Z. Fehér, J. Kiss, P. Kisszékelyi, J. Molnár, P. Huszthy, L. Kárpáti and J. Kupai, Green Chem., 2022, 24, 8447-8459.
3 A. Bahadoor, A. Brinkmann and J. E. Melanson, Anal. Chem., 2020, 93, 851-858.

### Embodiments

1. A method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a magnesium salt or calcium salt of a carboxylic acid having the formula: 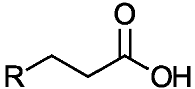 wherein R is selected from a C₆-C₂₀ aliphatic group, wherein R is optionally substituted with one or more: C₁-C₆ alkyl or C₁-C₆ alkoxy.
2. The method of embodiment 1, wherein R is a C₆-C₂₀ aliphatic group, optionally a C₆-C₂₀ alkyl group.
3. The method of embodiment 1 or 2, wherein R is a C₆-C₁₈ aliphatic group, optionally, C₇-C₁₈ aliphatic group, such as C₇-C₁₇ aliphatic group.
4. The method of any one of embodiments 1 to 3, wherein is a C₆-C₁₈ alkyl group, optionally, C₇-C₁₈ alkyl group, such as C₇-C₁₇ alkyl group.
5. The method of any preceding embodiment, wherein R is C₇-C₁₇ alkyl chain, optionally C₈-C₁₂ alkyl.
6. The method of any preceding embodiment, wherein the catalyst is a solid in ethylene glycol at room temperature.
7. The method of any preceding embodiment, wherein the catalyst comprises a magnesium salt.
8. The method of any one of embodiments 1 to 6, wherein the catalyst comprises a calcium salt.
9. The method of any preceding embodiment, wherein the catalyst comprises a magnesium salt or calcium salt of: nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).
10. The method of embodiment 8, wherein catalyst comprises a magnesium salt or calcium salt of: nonanoic acid (C₉), decanoic acid (C₁₀), undecanoic acid (C₁₁), dodecanoic acid (C₁₂), tridecanoic acid (C₁₃), and/or tetradecanoic acid (C₁₄).
11. The method of any preceding embodiment, wherein the catalyst comprises magnesium decanoate, magnesium undecanoate, magnesium dodecanoate, magnesium tridecanoate, magnesium tetradecanoate and/or magnesium stearate.
12. The method of any preceding embodiment wherein the catalyst is employed in an amount of 5 mol% or less, suitably in an amount of 2 mol% or less, more suitably, in an amount of 1 mol% or less relative, most suitably in an amount of 0.5 mol% or less per mole of monomeric repeating unit of polyethylene terephthalate.
13. The method of any preceding embodiment, wherein the weight ratio of PET:ethylene glycol is in the range of from 1:1 to 1:20, suitably in the range of from 1:2 to 1:15, preferably in the range of from 1:3 to 1:12.
14. The method of any preceding embodiment, wherein the catalyst comprises a mixture of two or more magnesium carboxylates having the formula: 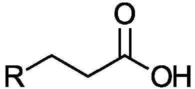 wherein R is selected from a C₆-C₂₀ aliphatic group, wherein R is optionally substituted with one or more: C₁-C₆ alkyl or C₁-C₆ alkoxy.
15. The method of embodiment 14, wherein the catalyst comprises two or more, optionally three or four or more of: magnesium salts of myristic acid, palmitic acid, lauric acid, stearic acid, oleic acid, linoleic acid.
16. The method of any preceding embodiment, further comprising a filtration step to remove the catalyst from a mixture comprising the BHET.
17. A method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a mixture of magnesium and/or calcium carboxylate salts formed from the hydrolysis of a plant oil or animal fat.
18. The method of embodiment 17, wherein the catalyst comprises one or more magnesium salts of: decanoic acid, palmitic acid, tetradecanoic acid, palmitic acid, stearic acid, oleic acid, linoleic acid and arachidonic acid.
19. The method of embodiment 17 or 18, wherein the plant oil is selected from vegetable oil, coconut oil, sunflower oil, peanut oil, rice oil, flaxseed oil, sesame oil, grape seed oil, rapeseed oil, olive oil, canola oil, and/or corn oil.
20. The method of any one of embodiments 17 to 19, wherein the mixture of magnesium carboxylate salts formed from the hydrolysis of a plant oil or animal fat includes a mixture of two or more magnesium salts of carboxylic acids including two or more of decanoic acid, myristic acid, palmitic acid, margaric acid, stearic acid, oleic acid, linoleic acid, linoleic acid and arachidic acid.
21. The method of any one of embodiments 17 to 19, wherein the plant oil or animal fat comprises waste cooking oil.
22. The method of any one of embodiments 17 to 20, wherein the catalyst is present in an amount of from 0.05 wt% to 5 wt% based on the weight of the polyethylene terephthalate, optionally from 0.1 wt% to 4 wt% based on the weight of the polyethylene terephthalate, further optionally from 0.1 to 1 wt% based on the weight of the polyethylene terephthalate.

## Claims

1. A method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a magnesium salt or calcium salt of a carboxylic acid having the formula: 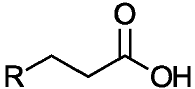 wherein R is selected from a C₆-C₂₀ aliphatic group, wherein R is optionally substituted with one or more: C₁-C₆ alkyl or C₁-C₆ alkoxy.

2. The method of claim 1, wherein R is a C₆-C₂₀ aliphatic group, optionally a C₆-C₂₀ alkyl group.

3. The method of any one of claims 1 or 2, wherein is a C₆-C₁₈ alkyl group, optionally, C₇-C₁₈ alkyl group, such as C₇-C₁₇ alkyl group.

4. The method of any preceding claim, wherein R is C₇-C₁₇ alkyl chain, optionally C₈-C₁₂ alkyl.

5. The method of any preceding claim, wherein the catalyst comprises a magnesium salt or calcium salt of: nonanoic acid (C9), decanoic acid (C₁₀), undecanoic acid (C₁₁), dodecanoic acid (lauric acid) (C₁₂), tridecanoic acid (C₁₃), tetradecanoic acid (myristic acid) (C₁₄), pentadecanoic acid (C₁₅), hexadecanoic acid (palmitic acid) (C₁₆), margaric acid (C₁₇), stearic acid (C₁₈), nonadecylic acid (C₁₉), arachidic acid (C₂₀), heneicosylic acid (C₂₁) and/or behenic acid (C₂₂).

6. The method of any preceding claim, wherein the catalyst comprises magnesium decanoate, magnesium undecanoate, magnesium dodecanoate, magnesium tridecanoate, magnesium tetradecanoate and/or magnesium stearate.

7. The method of any preceding claim wherein the catalyst is employed in an amount of 5 mol% or less, suitably in an amount of 2 mol% or less, more suitably, in an amount of 1 mol% or less relative, most suitably in an amount of 0.5 mol% or less per mole of monomeric repeating unit of polyethylene terephthalate.

8. The method of any preceding claim, wherein the weight ratio of PET:ethylene glycol is in the range of from 1:1 to 1:20, suitably in the range of from 1:2 to 1:15, preferably in the range of from 1:3 to 1:12.

9. The method of any preceding claim, wherein the catalyst comprises a mixture of two or more magnesium carboxylates having the formula: 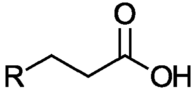 wherein R is selected from a C₆-C₂₀ aliphatic group, wherein R is optionally substituted with one or more: C₁-C₆ alkyl or C₁-C₆ alkoxy.

10. A method for preparing bis (2-hydroxyl) terephthalate (BHET) from a polyethylene terephthalate comprising: combining polyethylene terephthalate (PET), ethylene glycol and a catalyst and heating for a time sufficient to obtain BHET, wherein the catalyst comprises a mixture of magnesium and/or calcium carboxylate salts formed from the hydrolysis of a plant oil or animal fat.

11. The method of claim 10, wherein the catalyst comprises one or more magnesium salts of: decanoic acid, palmitic acid, tetradecanoic acid, palmitic acid, stearic acid, oleic acid, linoleic acid and arachidonic acid.

12. The method of claim 10 or 11, wherein the plant oil is selected from vegetable oil, coconut oil, sunflower oil, peanut oil, rice oil, flaxseed oil, sesame oil, grape seed oil, rapeseed oil, olive oil, canola oil, and/or corn oil.

13. The method of any one of claims 10 to 12, wherein the mixture of mixture of magnesium carboxylate salts formed from the hydrolysis of a plant oil or animal fat includes a mixture of two or more magnesium salts of carboxylic acids including two or more of decanoic acid, myristic acid, palmitic acid, margaric acid, stearic acid, oleic acid, linoleic acid, linoleic acid and arachidic acid.

14. The method of any one of claims 10 to 13, wherein the plant oil or animal fat comprises waste cooking oil.

15. The method of any one of claims 10 to 14, wherein the catalyst is present in an amount of from 0.05 wt% to 5 wt% based on the weight of the polyethylene terephthalate, optionally from 0.1 wt% to 4 wt% based on the weight of the polyethylene terephthalate, further optionally from 0.1 to 1 wt% based on the weight of the polyethylene terephthalate.
